# EUROPEAN PATENT APPLICATION

(11) **EP 2 592 416 A2**
(43) Date of publication of application: **15.05.2013**
(21) Application number: 12007568.4
(22) Date of filing: 07.11.2012
(51) Int. Cl.: G01N 33/00, F01N 3/20

(54) **Device for supplying simulated gas**

(30) Priority: 10.11.2011 JP 2011246867; 27.12.2011 JP 2011284885
(71) Applicant: HORIBA, LTD., Kyoto-shi, Kyoto 601-8510 (JP)
(72) Inventor: Okada, Yoichi, Kyoto, 601-8510 (JP); Shiota, Takahiro, Kyoto, 601-8510 (JP)
(74) Representative: Müller - Hoffmann & Partner

(57) **Abstract**

The present invention is one that, while suppressing a temperature variation of simulated gas supplied to a test target, changes a flow rate of the simulated gas supplied to the test target, and provided with: a first gas supply path 2; a first gas heating part 4 that is connected with the first gas supply path 2 and heats the first gas; a simulated gas supply path 6 that supplies the simulated gas generated by heating by the first gas heating part 4 to the test target; and a simulated gas discharge path 7 that, without supplying the simulated gas generated by heating by the first gas heating part 4 to the test target, discharges the simulated gas outside, wherein by controlling a discharge flow rate of the simulated gas through the simulated gas discharge path 7, a supply flow rate of the simulated gas supplied from the simulated gas supply path 6 to the test target is controlled.

## Description

### Technical field

The present invention relates to a simulated gas supply device that supplies simulated gas to a test object such as a catalyst or a gas analyzer.

### Background art

In the case of evaluating a catalyst provided in an exhaust pipe of a vehicle, or the like, simulated gas containing the same components as those of exhaust gas from an engine is conventionally used. Note that the simulated gas refers to gas in which various components contained in the engine exhaust gas, such as NOₓ, CO₂, HC, H₂, and O₂, are mixed with nitrogen gas so as to have the same concentrations as those in the engine exhaust gas.

Also, as an apparatus that uses the simulated gas to evaluate a catalyst, as disclosed in Patent literature 1, there is a catalyst evaluation test apparatus that is configured to be provided with: a first heating part and a second heating part in this order from an upstream side along a gas cell where simulated gas supplied from a simulated gas supply path flows; heat the simulated gas with the first heating part; and heat a catalyst installed in the gas cell with the second heating part.

The catalyst evaluation test apparatus is an apparatus that is configured to connect a branched flow path to the simulated exhaust gas supply path, and connect the branched flow path to the gas cell between the first heating part and the second heating part, and adjusts opening degrees of flow rate control valves respectively provided in the simulated gas supply path and the branched flow path to control temperature in the vicinity of an inlet of the catalyst to a predetermined temperature.

However, in the case of decreasing a flow rate of the simulated gas flowing into the first heating part with use of the flow rate control valve provided in the simulated gas supply path, there is a problem that a heat quantity provided from the first heating part to the simulated gas increases, and thereby the simulated gas temperature is made higher than a desired temperature. On the other hand, in the case of increasing the flow rate of the simulated gas flowing into the first heating part, there is another problem that the heat quantity provided from the first heating part to the simulated gas decreases, and thereby the temperature of the simulated gas is decreased. In addition, by controlling the first heating part, the temperature of the simulated gas in the vicinity of the inlet of the catalyst is controlled to address such problems; however, the temperature control has a response delay, and therefore it is difficult to do appropriate evaluation and testing.
Citation List
Patent Literature

[Patent literature 1] JPA 2003-126658

### Summary of Invention

### Technical Problem

Therefore, the present invention is made to solve the above problems at once, and a main intended object thereof is to, while suppressing a temperature variation of simulated gas supplied to a test object, change a flow rate of the simulated gas supplied to the test object.

### Solution to Problem

That is, a simulated gas supply device according to the present invention is a simulated gas supply device that generates simulated gas to supply the simulated gas to a test target, and provided with: a first gas supply path that supplies first gas constituting the simulated gas; a first gas heating part that is connected with the first gas supply path and heats the first gas supplied by the first gas supply path; a simulated gas supply path that supplies the simulated gas generated by heating the first gas in the first gas heating part to the test target; and a simulated gas discharge path that, without supplying the simulated gas generated by heating the first gas in the first gas heating part to the test target, discharges the simulated gas outside, wherein by controlling a discharge flow rate of the simulated gas through the simulated gas discharge path, a supply flow rate of the simulated gas supplied from the simulated gas supply path to the test target is controlled.

If so, without changing a flow rate of the first gas supplied to the first gas heating part, part of the simulated gas generated by heating the first gas in the first gas heating part is discharged outside to thereby control the flow rate of the simulated gas supplied to the test target, so that the flow rate of the simulated gas supplied to the test target can be varied with a temperature variation of the simulated gas supplied to the test target being suppressed. That is, even if the flow rate of the simulated gas supplied to the test target is changed, the flow rate of the first gas supplied to the first gas heating part can be made constant, and therefore the temperature variation of the simulated gas generated in the first gas heating part can be suppressed.

In order to generate a plurality of types of simulated gases in the simulated gas supply device, a plurality of gas supply paths that supplies the gases on a component basis are provided, and in each of the plurality of gas supply paths, a flow rate control device such as a flow rate control valve is provided. In this case, when supply flow rates of the simulated gases supplied to the test target such as a catalyst are controlled, setting flow rate values of the flow rate control mechanisms provided in the respective gas supply paths should be changed, so that it takes time until the flow rates are stabilized after the change, and therefore during the time, a concentration variation or a temperature variation occurs. Further, between the flow rate control devices provided in the gas supply paths and the simulated gas generating part such as a gas cell, pipe diameters and pipe lengths may be respectively different, and therefore a time lag occurs until the flow rates of the respective components supplied to the gas cell reach desired values, which also causes the occurrence of the concentration and temperature variations of the simulated gases.

A simulated gas supply device according to the present invention for solving this problem is a simulated gas supply device that generates simulated gas to supply the simulated gas to a test target, and provided with: a first gas supply path that supplied first gas constituting the simulated gas; a second gas supply path that supplies second gas constituting the simulated gas; a first gas heating part that is connected with the first gas supply path and heats the first gas supplied by the first gas supply path; a simulated gas generating part that is connected with the second gas supply path, and mixes the second gas supplied by the second gas supply path and the first gas heated by the first gas heating part to generate the simulated gas; a simulated gas supply path that supplies the simulated gas generated by the simulated gas generating part to the test target; and a simulated gas discharge path that, without supplying the simulated gas generated by the simulated gas generating part to the test target, discharges the simulated gas outside, wherein: by controlling a discharge flow rate of the simulated gas through the simulated gas discharge path, a supply flow rate of the simulated gas supplied from the simulated gas supply path to the test target is controlled.

If so, without changing a flow rate of the first gas supplied to the first gas heating part or a flow rate of the second gas supplied to the simulated gas generating part, part of the simulated gas generated by the simulated gas generating part is discharged outside to thereby control the flow rate of the simulated gas supplied to the test target, and therefore the flow rate of the simulated gas supplied to the test target can be varied with concentration and temperature variations of the simulated gas supplied to the test target being suppressed. That is, even if the flow rate of the simulated gas supplied to the test target is changed, the flow rate of the first gas supplied to the first gas heating part and the flow rate of the second gas supplied to the simulated gas generating part can be made constant, and therefore temperature and concentration variations of the simulated gas generated in the simulated gas generating part can be suppressed.

Desirably, the simulated gas discharge path has: a main discharge path provided with a first on/off valve and an exhaust pump sequentially from an upstream side; and an outside air introduction path that branches from between the first on/off valve and the exhaust pump in the main discharge path and is provided with an outside air introduction port and a second on/off valve sequentially from an upstream side. If so, by adjusting a valve opening degree of the first on/off valve and a valve opening degree of the second on/off valve, the discharge flow rate of the simulated gas discharged from the simulated gas generating part can be controlled.

Also, in this case, desirably, when the simulated gas is not discharged through the simulated gas discharge path, the first on/off valve is closed, and also the second on/off valve is opened; and when the simulated gas is discharged through the simulated gas discharge path, the first on/off valve is opened, and also the second on/off valve is closed. This enables, in a state where the exhaust pump is activated, the supply flow rate of the simulated gas to be immediately changed only by switching on/off between the first on/off valve and the second on/off valve.

In the case of using the first on/off valve and the second on/off valve with opening or closing both of the valves, control of the discharge flow rate of the simulated gas discharged through the simulated gas discharge path is performed by controlling a suction flow rate of the exhaust pump; however, this has a problem that it is not easy to discharge the simulated gas at an accurate flow rate. In this case, desirably, in the simulated gas discharge path, a flow rate control device such as a mass flow controller is provided. This enables the discharge flow rate to be accurately controlled.

Desirably, the simulated gas supply device is set up with a catalyst serving as the test target and has a catalyst heating part that heats the catalyst, wherein the catalyst heating part is connected with the simulated gas supply path.

On the other hand, in a conventional catalyst evaluation test apparatus, in a simulated gas supply path, a mass flow controller that controls a flow rate of simulated gas is arranged. Also, by controlling a setting flow rate of the mass flow controller, the flow rate of the simulated gas supplied to a first gas heating part is controlled.

However, in such an apparatus that controls (e.g., PID-controls) the flow rate of the simulated gas with the mass flow controller, due to a delay in control response of the mass flow controller, it is difficult to control a flow rate during a transient period of flow rate switching. That is, even in the case of performing flow rate control of the mass flow controller to achieve a desired flow rate change, there is a problem that, due to the delay in control response of the mass flow controller, a flow rate of the simulated gas actually generated does not meet the desired flow rate change.

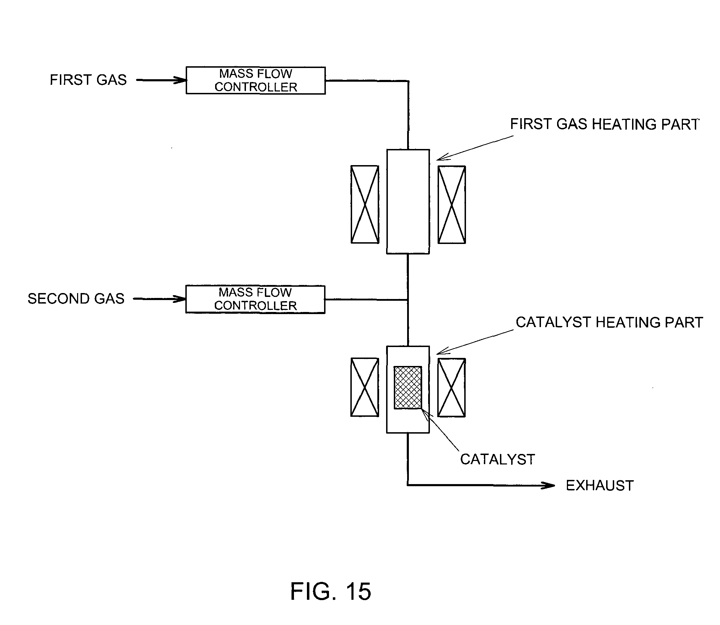

Also, in an apparatus that controls (e.g., PID-controls) a flow rate of simulated gas with a mass flow controller to control a concentration in the simulated gas, due to a delay in control response of the mass flow controller, it is difficult to control the component concentration during a transient period of concentration switching. That is, even in the case of performing flow rate control of the mass flow controller such that the component concentration in the simulated gas meets a desired concentration change, there is a problem that due to the delay in control response of the mass flow controller, the component concentration in the simulated gas actually generated does not meet the desired concentration change. This problem becomes particularly prominent in an apparatus that, as illustrated in Fig. 15, controls a plurality of mass flow controllers such as those respectively provided in a first gas supply path supplying first gas and in a second gas supply path supplying second gas, and controls a component concentration of simulated gas.

Therefore, a simulated gas supply device according to the present invention is a simulated gas supply device that generates simulated gas to supply the simulated gas to a test target, and provided with: a first gas supply path that supplies first gas for generating the simulated gas, the first gas being controlled to have a constant flow rate by a flow rate control device; a first gas heating part that is connected with the first gas supply path and heats the first gas supplied by the first gas supply path; and a simulated gas supply path that supplies the simulated gas generated by heating the first gas in the first gas heating part to the test target, wherein: the first gas supply path is branched into a first gas supply branch path that supplies the first gas to the first gas heating part and a first gas discharge branch path that discharges the first gas outside, and has a switching valve mechanism that selectively switches the first gas between the first gas supply branch path and the first gas discharge branch path; and by intermittently flowing the first gas to the first gas supply branch path with the switching valve mechanism, a flow rate of the first gas supplied to the first gas heating part is controlled.

If so, the first gas controlled to have the constant flow rate by the flow rate control device is selectively switched between the first gas supply branch path and the first gas discharge branch path, and thereby the first gas is intermittently flowed to the first gas supply branch path to control the flow rate of the first gas, so that it is not necessary to change the flow rate of the first gas with the flow rate control device. On the basis of this, it is only necessary for the flow rate control device to perform control such that the first gas has the constant flow rate, so that without taking into consideration a delay in control response of the flow rate control device, in the case of changing a flow rate of the simulated gas, control of the flow rate during a transient period of the change can be made easy, and the change desired for the flow rate of the simulated gas can be accurately reproduced. Also, as described, the control of the flow rate during the transient period can be made easy, so that in switching of a component concentration of the simulated gas, control of the component concentration during a transient period of the switching can be made easy, and a change desired for the component concentration of the simulated gas can be accurately reproduced.

Also, a simulated gas supply device according to the present invention is a simulated gas supply device that generates simulated gas to supply the simulated gas to a test target, and provided with: a first gas supply path that supplies first gas for generating the simulated gas; a second gas supply path that supplies second gas for generating the simulated gas, the second gas being controlled to have a constant flow rate by a flow rate control device; a first gas heating part that is connected with the first gas supply path and heats the first gas supplied by the first gas supply path; a simulated gas generating part that is connected with the second gas supply path, and mixes the second gas supplied by the second gas supply path and the first gas heated by the first gas heating part to generate the simulated gas; and a simulated gas supply path that supplies the simulated gas generated by the simulated gas generating part to the test target, wherein: the second gas supply path is branched into a second gas supply branch path that supplies the second gas to the simulated gas generating part and a second gas discharge branch path that discharges the second gas outside, and has a switching valve mechanism that selectively switches the second gas between the second gas supply branch path and the second gas discharge branch path; and by intermittently flowing the second gas to the second gas supply branch path with the switching valve mechanism, a flow rate of the second gas supplied to the simulated gas generating part is controlled.

If so, the second gas controlled to have the constant flow rate by the flow rate control device is switched between the second gas supply branch path and the second gas discharge branch path, and thereby the second gas is intermittently flowed to the second gas supply branch path to control the flow rate of the second gas supplied to the simulated gas generating part, so that it is not necessary to change the flow rate of the second gas with the flow rate control device. On the basis of this, it is only necessary for the flow rate control device to perform control such that the second gas has the constant flow rate, so that without taking into consideration a delay in control response of the flow rate control device, in the case of changing a flow rate of the simulated gas, control of the flow rate during a transient period of the change can be made easy, and the change desired for the flow rate of the simulated gas can be accurately reproduced. Also, as described, the control of the flow rate during the transient period can be made easy, so that in switching of a component concentration of the simulated gas, control of the component concentration during a transient period of the switching can be made easy, and a change desired for the component concentration of the simulated gas can be accurately reproduced.

In a simulated gas supply device provided with a plurality of first gas supply paths, the number of mass flow controllers is usually increased, and therefore the problem of the present invention becomes further prominent. For this reason, by applying the present invention to such a device, the effect of the present invention becomes further prominent. In addition, in the case where the plurality of first gas supply paths are provided, by making the types of first gases supplied by the respective first gas supply paths different, a plurality of types of simulated gases can be generated.

In a simulated gas supply device provided with a plurality of second gas supply paths, the number of mass flow controllers is usually increased, and therefore the problem of the present invention becomes further prominent. For this reason, by applying the present invention to such a device, the effect of the present invention becomes further prominent. In addition, in the case where the plurality of second gas supply paths are provided, by making the types of second gases supplied by the respective second gas supply paths different, a plurality of types of simulated gases can be generated.

### Advantageous Effects of Invention

According to the present invention configured as described, while a temperature variation of simulated gas supplied to a test object is suppressed, a flow rate of the simulated gas supplied to the test object can be changed.

### Brief Description of the Drawings

Fig. 1 is a schematic diagram illustrating a configuration of a simulated gas supply device in a first embodiment;
Fig. 2 is a schematic diagram illustrating a configuration of a simulated gas supply device in a variation;
Fig. 3 is a schematic diagram illustrating a configuration of a simulated gas supply device in a variation;
Fig. 4 is a schematic diagram illustrating a configuration of a simulated gas supply device in a variation;
Fig. 5 is a schematic diagram illustrating a configuration of a simulated gas supply device in a variation;
Fig. 6 is a schematic diagram illustrating a configuration of a simulated gas supply device in a variation;
Fig. 7 is a schematic diagram illustrating a configuration of a simulated gas supply device in a second embodiment;
Fig. 8 is a diagram illustrating a supply method and supply amounts of first and second gases in the same embodiment;
Fig. 9 is a schematic diagram illustrating a configuration of a simulated gas supply device in a variation;
Fig. 10 is a schematic diagram illustrating a configuration of a simulated gas supply device in a variation;
Fig. 11 is a schematic diagram illustrating a configuration of a simulated gas supply device in a variation;
Fig. 12 is a schematic diagram illustrating a configuration of a simulated gas supply device in a variation;
Fig. 13 is a schematic diagram illustrating a configuration of a simulated gas supply device in a variation;
Fig. 14 is a schematic diagram illustrating a configuration of a simulated gas supply device in a variation; and
Fig. 15 is a schematic diagram illustrating a configuration of a conventional simulated gas supply device.

### Description of Embodiments

In the following, a simulated gas supply device according to the present invention is described referring to the drawings.

### 1. First embodiment

A simulated gas supply device 100 of a first embodiment is one that generates simulated gas, which simulates engine exhaust gas, to supply the simulated gas to a catalyst 200 serving as a test target, and used with being incorporated in a catalyst evaluation test apparatus.

Specifically, the simulated gas supply device 100 is provided with: a first gas supply path 2 that supplies first gas constituting the simulated gas; a second gas supply path 3 that supplies second gas constituting the simulated gas; a first gas heating part 4 that is connected with the first gas supply path 2 and heats the first gas supplied by the first gas supply path 2; a simulated gas generating part 5 that is connected with the second gas supply path 3 and mixes the second gas supplied by the second gas supply path 3 and the first gas heated by the first gas heating part 4 to generate the simulated gas; a simulated gas supply path 6 that supplies the simulated gas generated by the simulated gas generating part 5 to the catalyst 200; and a simulated gas discharge path 7 that, without supplying the simulated gas generated by the simulated gas generating part 5 to the catalyst 200 discharges the simulated gas outside.

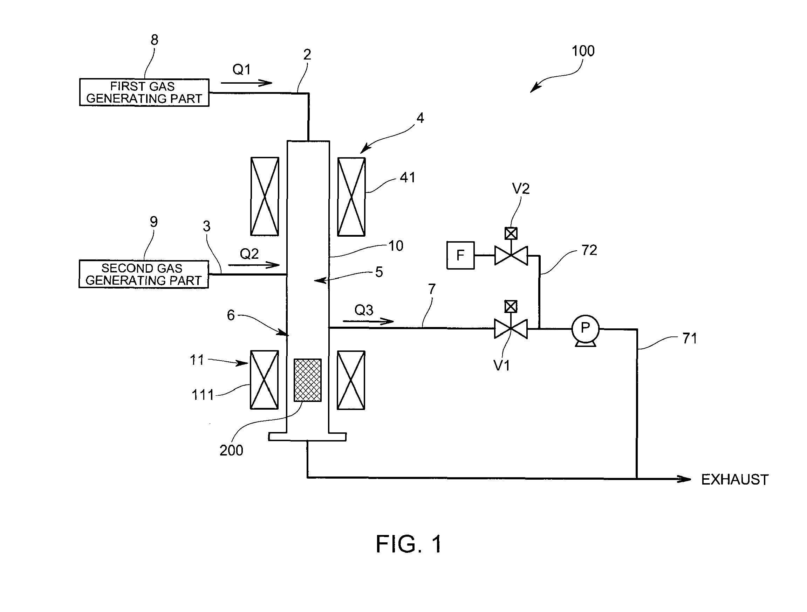

Fig. 1 illustrates only one first gas supply path 2; however, depending on components of the simulated gas, a plurality of first gas supply paths 2 are provided. Also, the first gas supply path 2 is provided with a first gas generating part 8 that generates the first gas. Note that the first gas may be mixed gas formed by mixing a plurality of component gases, or single component gas.

Fig. 1 also illustrates only one second gas supply path 3; however, depending on components of the simulated gas, a plurality of second gas supply paths 3 are provided. Further, the second gas supply path 3 is provided with a second gas generating part 9 that generates the second gas. The second gas is, for example, gas that reacts when being heated by the first gas heating part 4, and for example, CO, NOₓ, or the like. The second gas may also be, as with the first gas, mixed gas formed by mixing a plurality of component gases, or single component gas.

In the present embodiment, the first gas heating part 4, simulated gas generating part 5, and simulated gas supply path 6 are formed from a single chamber (simulated gas generating cell) 10. That is, in the simulated gas generating cell 10, the first gas heating part 4 is provided on an upstream side; on a downstream side, a catalyst heating part 11 is provided in order to heat the catalyst 200 arranged in the simulated gas generating cell 10; and between the first gas generating part 4 and the catalyst heating part 11, the simulated gas generating part 5 is provided. Also, the simulated gas generating part 5 is connected with the second gas supply path 3. Further, the simulated gas discharge path 7 is connected to the simulated gas generating part 5 or between the simulated gas generating part 5 and the catalyst heating part 11.

The first gas heating part 4 is configured to be provided with heating means 4 such as a heater in an outer circumferential part on an upstream side of the simulated gas generating cell 10. Also, the catalyst heating part 11 is configured to be provided with heating means 111 such as a heater in an outer circumferential part on the upstream side of the simulated gas generating cell 10. Further, the simulated gas generating part 5 is one that stirs the first and second gases to mix them, and, for example, may be one having a nozzle part.

In addition, the simulated gas having passed through the catalyst 200 in the catalyst heating part 11 is supplied to a gas analyzer (not illustrated) provided outside, where component concentrations in the simulated gas are analyzed. Also, by comparing the component concentrations with those of the simulated gas before passing through the catalyst, performance of the catalyst 200 is evaluated. In addition, in the vicinity of an inlet of the catalyst 200, a temperature detecting part (not illustrated) for detecting temperature of the catalyst 200 is provided, and on the basis of the detected temperature by the temperature detecting part, the temperature of the simulated gas is controlled by the catalyst heating part 11 to control the temperature of the catalyst 200.

The simulated gas supply device 100 of the first embodiment is configured to control a discharge flow rate of the simulated gas through the simulated gas discharge path 7 to thereby control a supply flow rate of the simulated gas supplied from the simulated gas supply path 6 to the catalyst 200.

Specifically, the simulated gas discharge path 7 has: a main discharge path 71 that is provided with a first one/off valve V1 and an exhaust pump P sequentially from an upstream side; and an outside air introduction path 72 that, in the main discharge path 71, branches from between the first on/off valve V1 and the exhaust pump P, and is provided with an outside air introduction port and a second on/off valve V2 sequentially from an upstream side. In addition, the outside air introduction port is provided with a dust filter F. Each of the first on/off valve V1 and the second on/off valve V2 of the present embodiment is a solenoid valve, and on/off thereof is controlled by a controller (not illustrated).

Specifically, in the case of not discharging the simulated gas through the simulated gas discharge path 7, control is performed so as to close the first on/off valve V1, and open the second on/off valve V2. On the other hand, in the case of discharging the simulated gas through the simulated gas discharge path 7, the first on/off valve V1 is opened, and also the second on/off valve V2 is closed. Assuming here that a first gas supply amount to the simulated gas generating cell 10 in the first gas supply path 2 is Q1, and a second gas supply amount to the simulated gas generating cell 10 in the second gas supply path 3 is Q2, in the case where an exhaust flow rate (Q3) of the exhaust pump P is constant, by performing control as described above, the supply flow rate of the simulated gas supplied to the catalyst 200 is controlled in two stages of a full supply (Q1 + Q2) and a partial supply (Q1 + Q2 - Q3).

According to the simulated gas supply device 100 according to the first embodiment configured as described, even if the supply flow rate of the simulated gas supplied to the catalyst 200 is changed by controlling the exhaust flow rate (Q3) of the simulated gas, the flow rate (Q1) of the first gas supplied to the first gas heating part 4 and the flow rate (Q2) of the second gas supplied to the simulated gas generating part 5 can be made constant. Accordingly, the flow rate of the simulated gas supplied to the catalyst 200 can be changed with concentration and temperature variations of the simulated gas supplied to the catalyst 200 being suppressed.

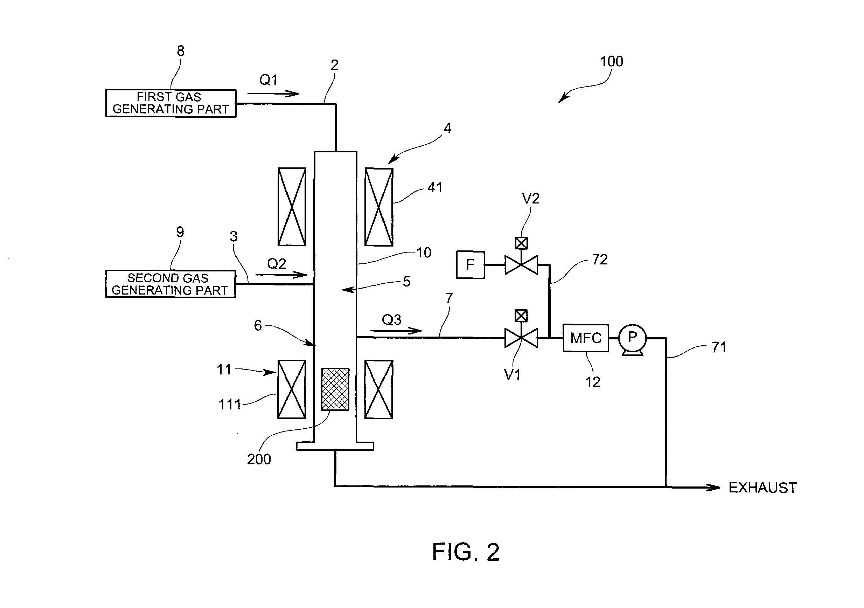

Note that the present invention is not limited to the above-described first embodiment.
For example, as illustrated in Fig. 2, the present invention may be one that, by providing a flow rate control device 12 such as a mass flow controller on an upstream side of the exhaust pump P in the simulated gas discharge path 7, continuously changes the exhaust flow rate through the simulated gas discharge path 7. This enables the supply flow rate of the simulated gas to the catalyst to be continuously changed with the temperature and concentration variations of the simulated gas being suppressed.

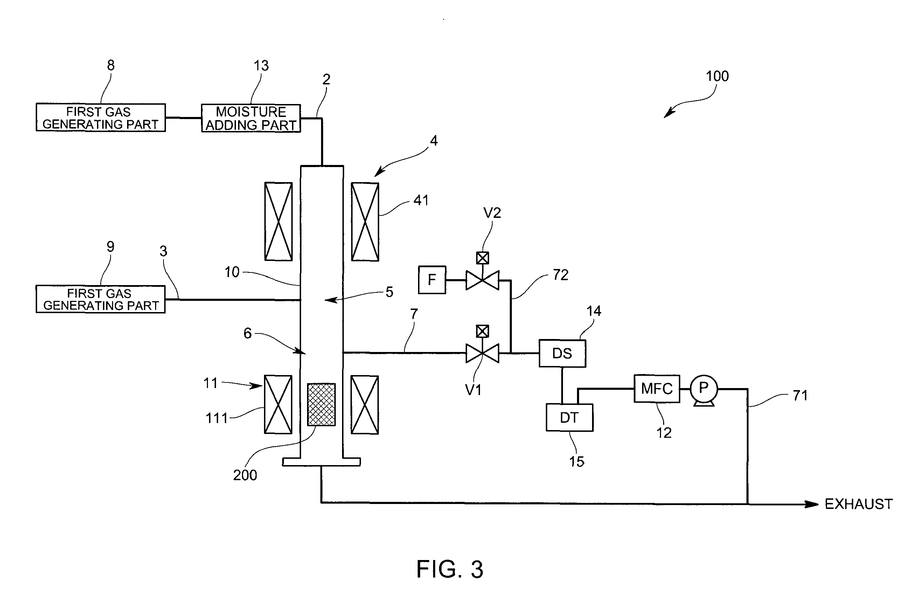

Also, as illustrated in Fig. 3, in order to soak the simulated gas with moisture, the first gas supply path 2 may be provided with a moisture adding part 13. In this case, the simulated gas discharge path 7 (main discharge path 71) is desirably provided with: a drain separator 14 that separates moisture in the simulated gas to be discharged; and a drain tank 15 that accumulates the moisture.

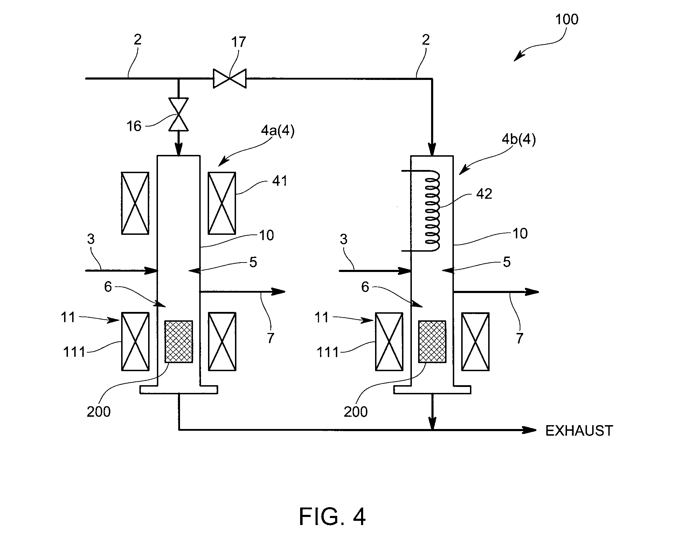

Further, the first gas heating part 4 of the above-described first embodiment is one that provides the outside of the chamber 10 with the heating means 41 such as a heater to thereby heat the first gas inside the chamber 10; however, as illustrated in Fig. 4, the first gas heating part 4 may be one that provides the inside of the chamber 10 with a heating resistive element 42, and thereby directly heats the first gas with the resistive element 42 being in contact with the first gas. This enables responsiveness of temperature control of the first gas to be improved. In addition, in Fig. 4, the present invention is configured to provide a first gas heating part 4 (4a) providing the outside of the chamber 10 with the heating means 41 such as a heater and a first gas heating part 4 (4b) providing the inside of the chamber 10 with the heating resistive element 42 in parallel, and be able to select any of them with switching valves 16 and 17.

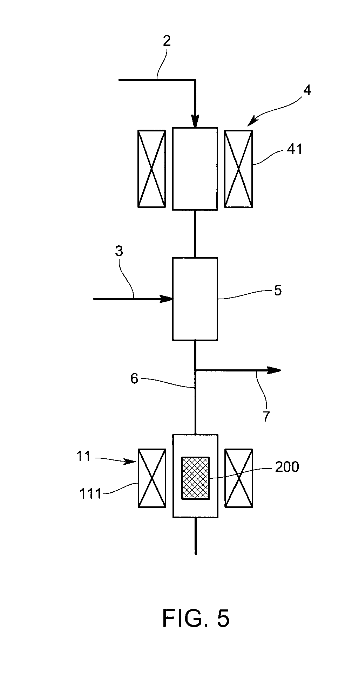

In addition, in the above-described first embodiment, the first gas heating part 4, simulated gas generating part 5, and simulated gas supply path 6 are integrally formed from the simulated gas generating cell; however, as illustrated in Fig. 5, the first gas heating part 4, simulated gas generating part 5, and simulated gas supply path 6 may be separately formed. In this case, the first gas heating part 4 and the simulated gas generating part 5 are connected to each other through a connecting pipe; the simulated gas generating part 5 is connected with the simulated gas supply path 6; and the simulated gas supply path 6 or the simulated gas generating part 5 is connected with the simulated gas discharge path 7. Also, the simulated gas supply path 6 is connected to the catalyst heating part 11 that heats the catalyst 200.

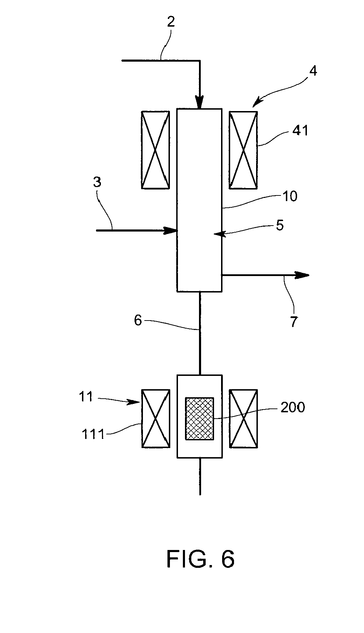

Still in addition, as illustrated in Fig. 6, the present invention may be configured to form the first gas heating part 4 and simulated gas generating part 5 from the simulated gas generating cell 10, and connect the simulated gas supply path 6 and simulated gas discharge path 7 to the simulated gas generating cell 10.

Also, the above-described first embodiment has one first gas supply path and one second gas supply path; however, besides, the present invention may have pluralities of first gas supply paths and second gas supply paths. In this case, the type of gas to be supplied may be made different for each of the plurality of provided first gas supply paths. In the same manner, the type of gas to be supplied may be made different for each of the plurality of provided second gas supply paths.

Further, in the above-described first embodiment, the simulated gas is generated by mixing the first gas and the second gas; however, the simulated gas may be one that is generated by heating the first gas in the first gas heating part without using the second gas.

### 2. Second embodiment

Next, a simulated gas supply device X100 of a second embodiment is described. The simulated gas supply device X100 of the second embodiment is also one that, as with the above-described first embodiment, generates simulated gas, which simulates engine exhaust gas, to supply the simulated gas to a catalyst 200 serving as a test object, and used with being incorporated in a catalyst evaluation test apparatus.

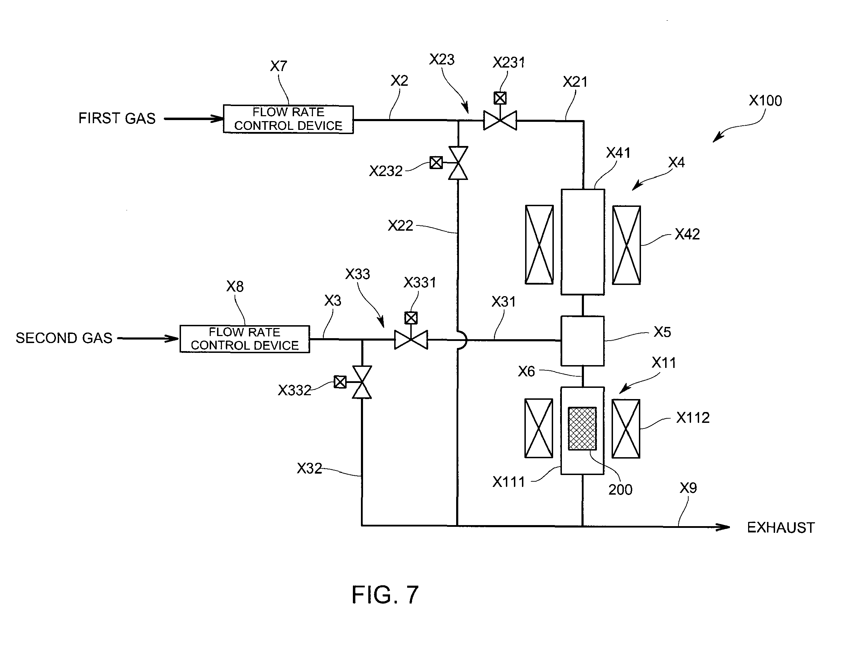

Specifically, the simulated gas supply device X100 is, as illustrated in Fig. 7, provided with: a first gas supply path X2 that supplies first gas for generating the simulated gas; a second gas supply path X3 that supplies second gas for generating the simulated gas; a first gas heating part X4 that is connected with the first gas supply path X2 and heats the first gas supplied by the first gas supply path X2; a simulated gas generating part X5 that is connected with the second gas supply path X3, and mixes the second gas supplied by the second gas supply path X3 and the first gas heated by the first gas heating part X4 to generate the simulated gas; and a simulated gas supply path X6 that supplies the simulated gas generated by the simulated gas generating part X5 to the catalyst 200.

Fig. 7 illustrates only one first gas supply path X2; however, depending on components of the first gas, a plurality of first gas supply paths X2 may be provided. Also, the first gas supply path X2 is connected with a first gas generating part (not illustrated) that generates the first gas. Note that the first gas may be mixed gas formed by mixing a plurality of component gases, or single component gas.

Further, the first gas supply path X2 is provided with a flow rate control device X7 that is intended to control a flow rate of the first gas supplied from the first gas generating part to a constant flow rate. The flow rate control device X7 may be configured to include, for example, a mass flow controller; however, in the present embodiment, by setting a flow rate setting value of the mass flow controller to a constant, the flow rate of the first gas flowing through the first gas supply path X2 is controlled to a constant flow rate. In addition, as the flow rate control device X7, besides, a flow rate control valve or the like may be used.

Fig. 7 also illustrates only one second gas supply path X3; however, depending on components of the second gas, a plurality of second gas supply paths 3 may be provided. Further, the second gas supply path X3 is connected with a second gas generating part (not illustrated) that generates the second gas. The second gas is gas that reacts when being heated by the first gas heating part X4, and for example, CO, NOₓ, or the like. The second gas may also be, as with the first gas, mixed gas formed by mixing a plurality of component gases, or single component gas.

Also, the second gas supply path X3 is provided with a flow rate control device X8 that is intended to control a flow rate of the second gas supplied from the second gas generating part to a constant flow rate. The flow rate control device X8 may be configured to include, for example, a mass flow controller; however, in the present embodiment, by setting a flow rate setting value of the mass flow controller to a constant, the flow rate of the first gas flowing through the second gas supply path X3 is controlled to a constant flow rate. In addition, as the flow rate control device X8, besides, a flow rate control valve or the like may be used.

The first gas heating part X4 is provided with: a cell X41 that is supplied with the first gas by the first gas supply path X2; and heating means X42 such as a heater provided in an outer circumferential part of the cell X41. Also, a catalyst heating part X11 is provided with: a cell X111 that contains the catalyst serving as the test object, and is also supplied with the simulated gas generated by the simulated gas generating part X5; and heating means X112 such as a heater provided in an outer circumferential part of the cell X111. Further, the simulated gas generating part X5 is one that stirs the first and second gases to mix them, and for example, may be one having a nozzle part.

In addition, the simulated gas having passed through the catalyst 200 in the catalyst heating part X11 is supplied to a gas analyzer (not illustrated) provided outside, where component concentrations in the simulated gas are analyzed. Also, by comparing the component concentrations with those of the simulated gas before passing through the catalyst, performance of the catalyst 200 is evaluated. In addition, in the vicinity of an inlet of the catalyst 200, a temperature detecting part (not illustrated) for detecting temperature of the catalyst 200 is provided, and on the basis of the detected temperature by the temperature detecting part, the catalyst heating part X11 is controlled to control the temperature of the catalyst 200.

Also, in the simulated gas supply device X100 of the second embodiment, the first gas supply path X2 is branched into: a first gas supply branch path X21 that supplies the first gas to the first gas heating part X4; and a first gas discharge branch path X22 that discharges the first gas outside, and has a first gas switching valve mechanism X23 that selectively switch the first gas between the first gas supply branch path X21 and the first gas discharge branch path X22.

The first gas supply branch path X21 and the first gas discharge branch path X22 branch on a downstream side of the flow rate control device X7. Also, the first gas supply branch path X21 is connected to the first gas heating part X4. Further, the first gas discharge branch path X22 is connected to an exhaust path X9 connected to the catalyst heating part X11.

The first gas switching valve mechanism X23 has: a supply side on/off valve X231 provided in the first gas supply branch path X21; and a discharge side on/off valve X232 provided in the first gas discharge branch path X22. Each of these on/off valves X231 and X232 is a solenoid valve that is openable and closable by, for example, milliseconds, and an on/off time thereof is controlled by a controller. In addition, the controller (not illustrated) is a dedicated or general-purpose computer having a CPU, a memory, an input/output interface, an AC converter, and the like.

Also, the second gas supply path X3 is branched into: a second gas supply branch path X31 that supplies the second gas to the simulated gas generating part X5; and a second gas discharge branch path X32 that discharges the second gas outside, and has a second gas switching valve mechanism X33 that selectively switches the second gas between the second gas supply branch path X31 and the second gas discharge branch path X32.

The second gas supply branch path X31 and the second gas discharge branch path X32 branch on a downstream side of the flow rate control device X8. Also, the second gas supply branch path X31 is connected to the simulated gas generating part X5. Further, the second gas discharge branch path X32 is connected to the exhaust path X9 connected to the catalyst heating part X11.

The second gas switching valve mechanism X33 has: a supply side on/off valve X331 provided in the second gas supply branch path X31; and a discharge side on/off valve X332 provided in the second gas discharge branch path X32. Each of these on/off valves X331 and X332 is a solenoid valve that is openable and closable by, for example, milliseconds, and an open/close time thereof is controlled by the controller.

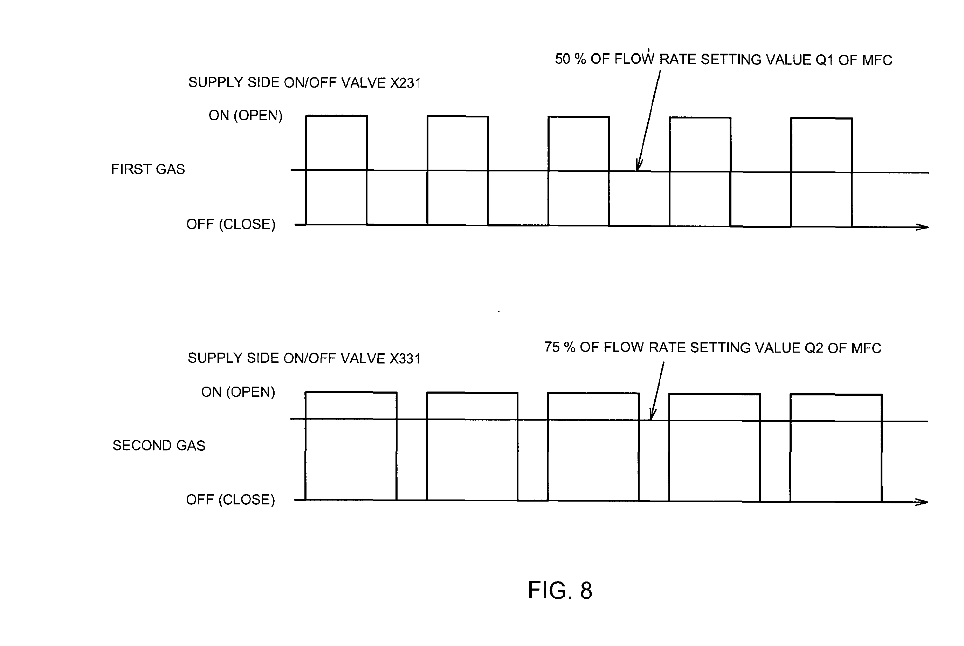

Next, operation of the simulated gas supply device X100 configured as above is described referring to Fig. 8.

The controller controls the first gas switching valve mechanism X23 to thereby selectively switch the first gas, which is controlled to have a constant flow rate by the flow rate control device X7, between the first gas supply branch path X21 and the first gas discharge branch path X22, and intermittently flows the first gas to the first gas supply branch path X21 to thereby control the flow rate of the first gas supplied to the first gas heating part X4. Specifically, the controller performs on/off control of the supply side on/off valve X231 and discharge side on/off valve X232 of the first gas switching valve mechanism X23. For example, as illustrated in an upper part of Fig. 8, by setting an on/off time ratio (duty ratio) of the supply side on/off valve X231 of the first gas switching valve mechanism X23 to 50 %, a flow rate equal to 50 % of a flow rate setting value Q1 of the flow rate control device X7 can be supplied to the first gas heating part X4. As described, by controlling the on/off time ratio of the supply side on/off valve X231, the flow rate supplied to the first gas heating part X4 is controlled. In addition, in the case of closing the supply side on/off valve X231, the discharge side on/off valve X232 is opened to discharge the first gas.

Also, the controller controls the second gas switching valve mechanism X33 to thereby selectively switch the second gas, which is controlled to have a constant flow rate by the flow rate control device X8, between the second gas supply branch path X31 and the second gas discharge branch path X32, and intermittently flows the second gas to the second gas supply branch path X31 to thereby control the flow rate of the second gas supplied to the simulated gas generating part X5. Specifically, the controller performs on/off control of the supply side on/off valve X331 and discharge side on/off valve X332 of the second gas switching valve mechanism X33. For example, as illustrated in a lower part of Fig. 8, by setting an on/off time ratio (duty ratio) of the supply side on/off valve X331 of the second gas switching valve mechanism X33 to 75 %, a flow rate equal to 75 % of a flow rate setting value Q2 of the flow rate control device X8 can be supplied to the simulated gas generating part X5. As described, by controlling the on/off time ratio of the supply side on/off valve X331, the flow rate supplied to the simulated gas generating part X5 is controlled. In addition, in the case of closing the supply side on/off valve X331, the discharge side on/off valve X332 is opened to discharge the second gas.

According to the simulated gas supply device X100 according to the second embodiment configured as described, by selectively switching the first gas, which is controlled to have a constant flow rate by the flow rate control deice X7, between the first gas supply branch path X21 and the first gas discharge branch path X22, the first gas is intermittently flowed to the first gas supply branch path X21 to control the flow rate of the first gas supplied to the first gas heating part X4, and therefore it is not necessary to change the flow rate of the first gas with the flow rate control device X7. Also, by switching the second gas, which is controlled to have a constant flow rate by the flow rate control deice X8, between the second gas supply branch path X31 and the second gas discharge branch path X32, the second gas is intermittently flowed to the second gas supply branch path X31 to control the flow rate of the second gas supplied to the simulated gas generating part X5, and therefore it is not necessary to change the flow rate of the second gas with the flow rate control device X8. On the basis of this, it is only necessary for the flow rate control devices X7 and X8 to perform control such that the first gas and the second gas have the constant flow rates, so that without taking into consideration delays in control responses of the flow rate control devices X7 and X8, in the case of changing a flow rate of the simulated gas, control of the flow rate during a transient period of the change can be made easy, and the change desired for the flow rate of the simulated gas can be accurately reproduced. Also, the control of the flow rate during the transient period can be made easy, so that in switching of component concentrations of the simulated gas, control of the component concentrations during a transient period of the switching can be made easy, and a change desired for the component concentrations of the simulated gas can be accurately reproduced.

Note that the present invention is not limited to the above-described embodiment.

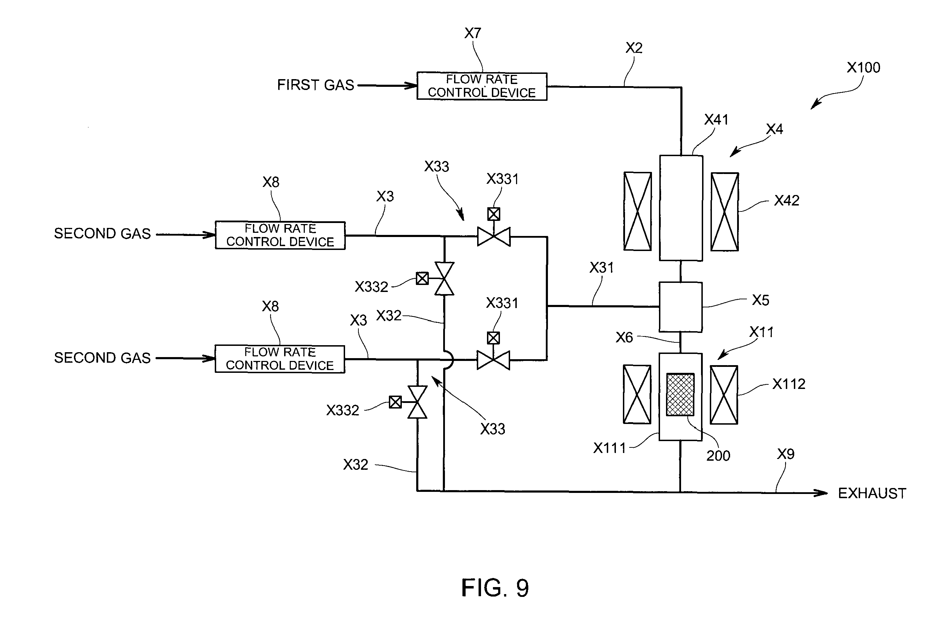

For example, as illustrated in Fig. 9, the present invention may be provided with a plurality of (in Fig. 9, two) second gas supply paths X3 that supply the second gas to the simulated gas generating part X5. In this case, the types of the second gases supplied by the respective second gas supply paths X3 to the simulated gas generating part X5 may be made different from each other. Note that making the types of the respective second gases different from each other refers to, in the case where each of the second gases includes a single component, making a component of one of the second gases different from that of the other second gas, and in the case where each of the second gases is mixed gas formed by mixing a plurality of component gases, making a plurality of components constituting one of the second gases different from those constituting the other second gas partially or wholly. In addition, Fig. 9 illustrates the case where, as before, in the first gas supply path X2, the flow rate of the first gas is controlled by the flow rate control device X7.

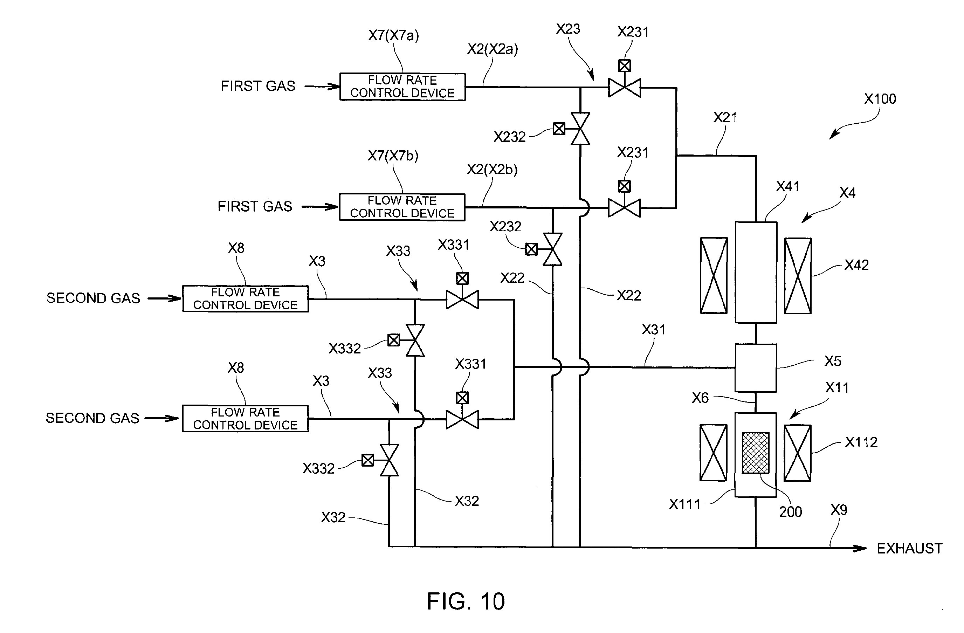

Also, as illustrated in Fig. 10, the present invention may be provided with a plurality of (in Fig. 10, two) first gas supply paths X2 that supply the first gas to the first gas heating part X4, and also provided with a plurality of (in Fig. 10, two) second gas supply paths X3 that supply the second gas to the simulated gas generating part X5. In this case, the types of the first gases supplied by the respective first gas supply paths X2 to the first gas heating part X4 may be made different from each other. Note that making the types of the respective first gases different from each other refers to, in the case where each of the first gases includes a single component, making a component of one of the first gases different from that of the other first gas, and in the case where each of the first gases is mixed gas formed by mixing a plurality of component gases, making a plurality of components constituting one of the first gases different from those constituting the other first gas partially or wholly.

Further, as illustrated in Figs. 9 and 10, in the case where the plurality of first gas supply paths X2 or second gas supply paths X3 are provided, the present invention may be configured to make flow rate setting values of flow rate control devices X7 or X8 provided in the gas supply paths X2 or X3 different from each other. For example, in the case of providing two first gas supply paths X2, and supplying the same first gas through the first gas supply paths X2, as illustrated in Fig. 10, assuming that a flow rate setting value of the flow rate control device X7a provided in one of the first gas supply paths X2a is Qx, and a flow rate setting value of the flow rate control device X7b provided in the other first gas supply path X2b is Qy, the present invention may set Qx and Qy to meet, for example Qx > Qy. As described, if the present invention is one that uses the plurality of first gas supply paths X2 to make the setting flow rate values of the corresponding flow rate control devices X7 different from each other, as compared with the case of using one first gas supply path X2 to perform flow rate control on the basis of the on/off control by the switching valve mechanism X23 in the first gas supply path X2, the flow rate control of the first gas can be performed over a wider range.

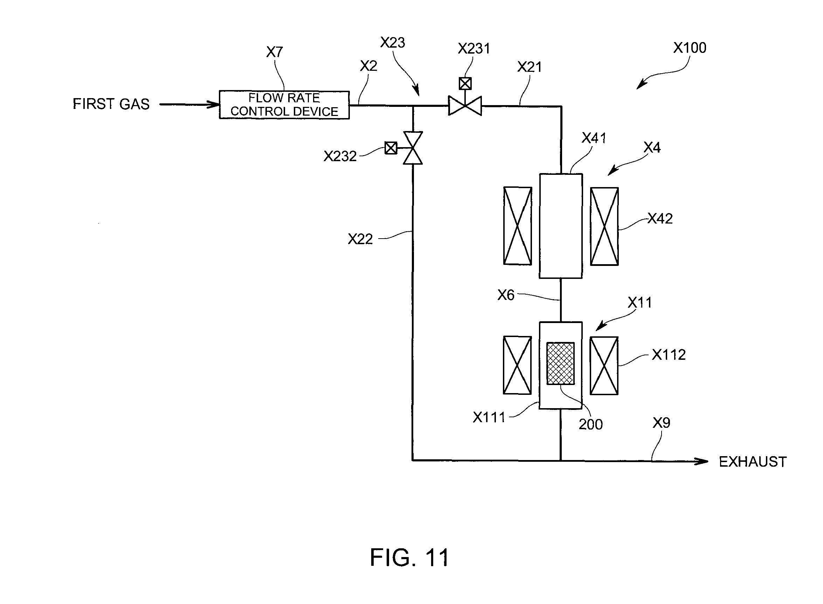

Further, in the above-described embodiment, the simulated gas is generated by mixing the first gas and the second gas; however, as illustrated in Fig. 11, the simulated gas may be one that is, without using the second gas, generated by heating the first gas in the first gas heating part. In this case, in switching of a flow rate of the simulated gas, control of the flow rate during a transient period of the switching can be made easy, and a change desired for the flow rate of the simulated gas can be accurately reproduced.

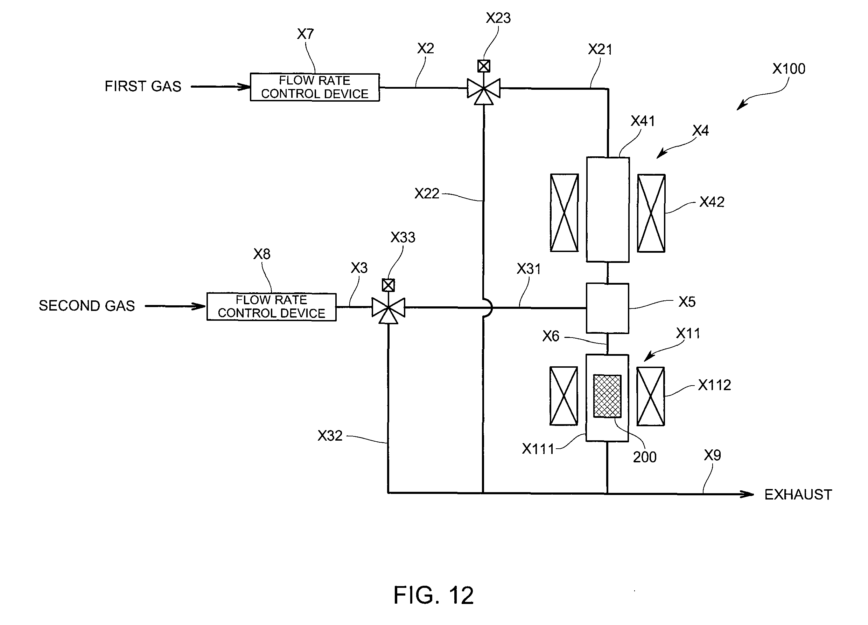

In addition, as the switching valve mechanism X23 or X33, in addition to a switching valve mechanism including the supply side on/off valve X231 or X331 and the discharge side on/off valve X232 or X332, as illustrated in Fig. 12, a switching valve mechanism configured with use of a three-way valve is also possible. This enables a piping configuration to be simplified.

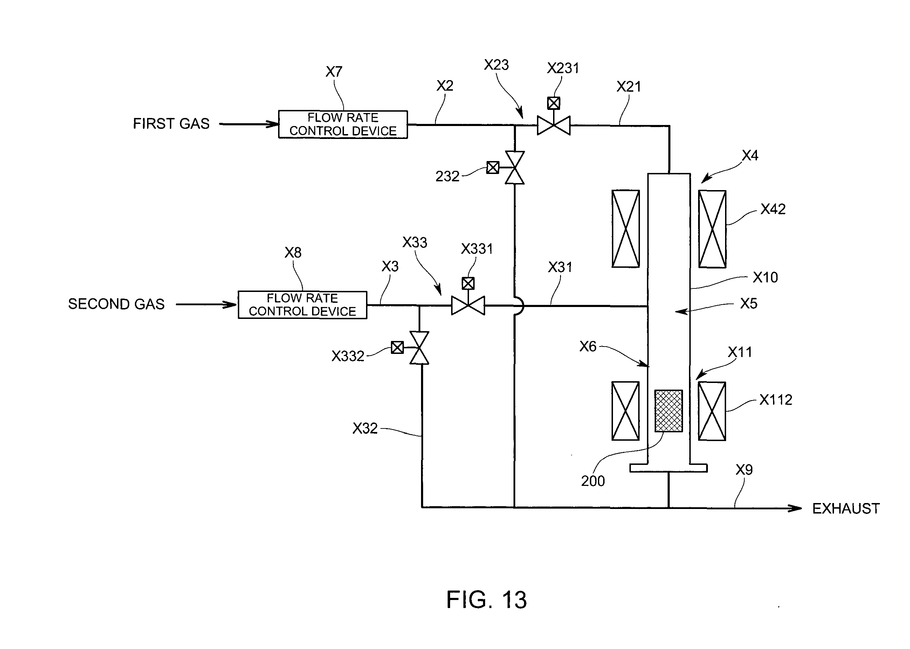

Also, in the above-described embodiment, the first gas heating part X4, simulated gas generating part X5, simulated gas supply path X6, and catalyst heating part X11 are separately configured; however, as illustrated in Fig. 13, the first gas heating part X4, simulated gas generating part X5, simulated gas supply path X6, and catalyst heating part X11 may be formed from a single chamber (simulated gas generating cell) X10. That is, in the simulated gas generating cell X10, on an upstream side, the first gas heating part X4 is provided; on a downstream side, the catalyst heating part X11 for heating the catalyst 200 arranged in the simulated gas generating cell X10 is provided; and between the first gas heating part X4 and the catalyst heating part X11, the simulated gas generating part X5 is provided. Also, the simulated gas generating part X5 is connected with the second gas supply path X3.

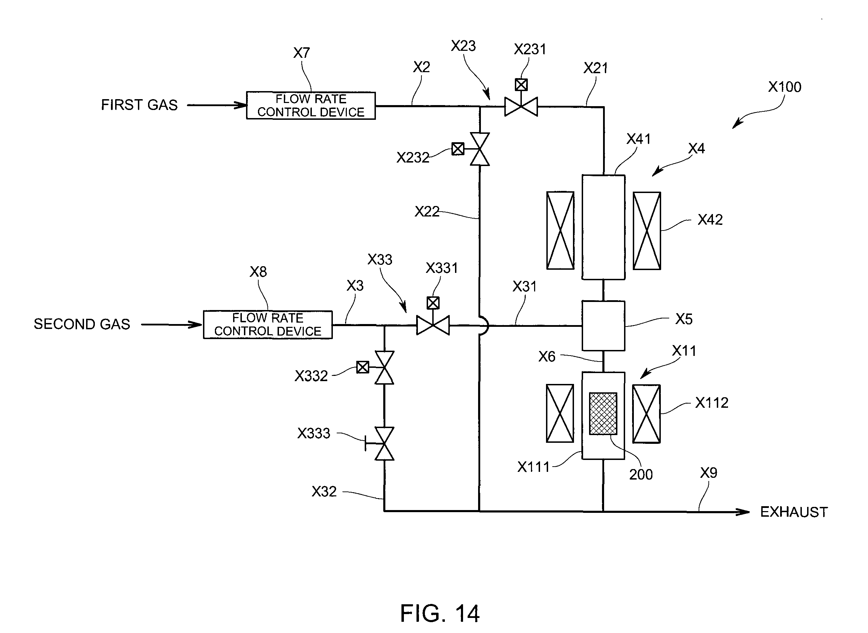

Further, in the simulated gas supply device X100 of the above-described embodiment, as illustrated in Fig. 14, on a downstream side of the discharge side on/off valve X332 in the second gas discharge branch path X32, a pressure regulating mechanism X333 such as a pressure regulating valve is desirably provided. The pressure regulating mechanism X333 is one that regulates pressure of an outlet port of the discharge side on/off valve X332, and specifically, regulates the pressure of the outlet port of the discharge side on/off valve X332 to the same pressure as pressure of an outlet port of the supply side on/off valve X331 in the second gas supply branch path X31. More specifically, a pressure sensor is provided on the outlet port side of the supply side on/off valve X331, and the controller obtained a pressure signal from the pressure sensor controls the pressure regulating mechanism X333 to thereby regulate the pressure of the outlet port of the discharge side on/off valve X332 to the same pressure as the pressure of the outlet port of the supply side on/off valve X331 in the second gas supply branch path X31. This enables the gas to be stably supplied from the second gas supply branch path X31 to the simulated gas generating part X5 and the catalyst heating part X11. In addition, in the same manner, on a downstream side of the discharge side on/off valve X232 in the first gas discharge branch path X22, a pressure regulating mechanism such as a pressure regulating valve may also be provided.

Besides, in addition to being used for the catalyst evaluation test apparatus, the present invention can also be applied to an apparatus doing a performance test of a gas analyzer by supplying the simulated gas to the gas analyzer.

In addition, it should be appreciated that the present invention is not limited to any of the above-described embodiments, but can be variously modified without departing from the scope thereof.

### Reference Signs List

100: Simulated gas supply device
200: Catalyst
2: First gas supply path
3: Second gas supply path
4: First gas heating part
5: Simulated gas generating part
6: Simulated gas supply path
7: Simulated gas discharge path
71: Main discharge path
V1: First on/off valve
P: Exhaust pump
72: Outside air introduction path
V2: Second on/off valve
11: Catalyst heating part
12: Flow rate control device

## Claims

1. A simulated gas supply device that generates simulated gas to supply the simulated gas to a test target, the simulated gas supply device comprising:
a first gas supply path that supplies first gas constituting the simulated gas;
a first gas heating part that is connected with the first gas supply path and heats the first gas supplied by the first gas supply path;
a simulated gas supply path that supplies the simulated gas generated by heating the first gas in the first gas heating part to the test target; and
a simulated gas discharge path that, without supplying the simulated gas generated by heating the first gas in the first gas heating part to the test target, discharges the simulated gas outside, wherein
by controlling a discharge flow rate of the simulated gas through the simulated gas discharge path, a supply flow rate of the simulated gas supplied from the simulated gas supply path to the test target is controlled.

2. The simulated gas supply device according to claim 1, further comprising:
a second gas supply path that supplies second gas constituting the simulated gas; and
a simulated gas generating part that is connected with the second gas supply path, and mixes the second gas supplied by the second gas supply path and the first gas heated by the first gas heating part to generate the simulated gas, wherein :
the simulated gas supply path supplies the simulated gas generated by the simulated gas generating part to the test target; and
the simulated gas discharge path discharges, without supplying the simulated gas generated by the simulated gas generating part to the test target, the simulated gas outside.

3. The simulated gas supply device according to claim 1, wherein
the simulated gas discharge path has:
a main discharge path provided with a first on/off valve and an exhaust pump sequentially from an upstream side; and
an outside air introduction path that branches from between the first on/off valve and the exhaust pump in the main discharge path and is provided with an outside air introduction port and a second on/off valve sequentially from an upstream side.

4. The simulated gas supply device according to claim 3, wherein:
when the simulated gas is not discharged through the simulated gas discharge path, the first on/off valve is closed, and also the second on/off valve is opened; and
when the simulated gas is discharged through the simulated gas discharge path, the first on/off valve is opened, and also the second on/off valve is closed.

5. The simulated gas supply device according to claim 1, wherein
in the simulated gas discharge path, a flow rate control device is provided.

6. The simulated gas supply device according to claim 1, the simulated gas supply device being set up with a catalyst serving as the test target and having a catalyst heating part that heats the catalyst, wherein
the catalyst heating part is connected with the simulated gas supply path.
